# EUROPÄISCHE PATENTSCHRIFT

(11) **EP 0 827 955 B1**
(45) Veröffentlichungstag und Bekanntmachung des Hinweises auf die Patenterteilung: **25.02.2004**
(21) Anmeldenummer: 97114194.0
(22) Anmeldetag: 18.08.1997
(51) Int. Cl.: C07D 233/54

(54) **Verfahren zur Herstellung substituierter Imidazole**
Process for the preparation of substituted imidazoles
Procédé pour la préparation d'imidazoles substituées

(30) Priorität: 19.08.1996 DE 19633390
(43) Veröffentlichungstag der Anmeldung: 11.03.1998
(73) Patentinhaber: BASF AKTIENGESELLSCHAFT, 67056 Ludwigshafen (DE)
(72) Erfinder: Hieber, Gisela, Dr., 69121 Heidelberg (DE); Ebel, Klaus, Dr., 68623 Lampertheim (DE); Schröder, Jürgen, Dr., 67071 Ludwigshafen (DE); Dockner, Toni, Dr., 67149 Meckenheim (DE); Gröning, Carsten, Dr., 68259 Mannheim (DE); Ruge, Bernd, Dr., 67459 Böhl-Iggelheim (DE)
(74) Vertreter: Isenbruck, Günter, Dr.

(56) Entgegenhaltungen:
- DE-A- 1 670 293
- DE-A- 2 106 877
- DE-A- 2 233 908
- TANAKA N ET AL: "Transition Metal-Catalyzed N-Alkylation of NH Grouips of Azoles with Alcohols" CHEMISTRY LETTERS., Nr. 4, April 1992, TOKYO JP, Seiten 575-578, XP002047812

## Beschreibung

Die Erfindung betrifft ein Verfahren zur Herstellung von substituierten Imidazolen der allgemeinen Formel I 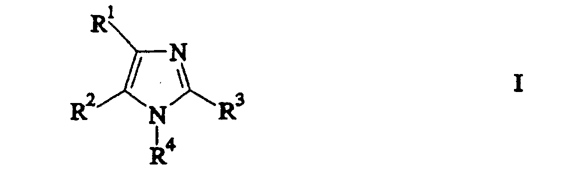 worin
R¹, R² und R³ gleich oder verschieden sein können und jeweils Wasserstoff, Halogen, NO₂, CN oder einen gegebenenfalls mit einem oder mehreren Halogenen substituierten C₁- bis C₂₀-Kohlenwasserstoffrest bedeuten oder R¹ und R² zusammen Glieder eines gegebenenfalls mit einem oder mehreren Halogenen und/oder C₁- bis C₈-Alkyl substituierten 3 bis 20 gliedrigen Kohlenwasserstoffringes bilden, der gegebenenfalls 1, 2 oder 3 Herteroatome aus der Gruppe Stickstoff, Sauerstoff und Schwefel aufweist, und
R⁴ einen C₁- bis C₂₀-Kohlenwasserstoffrest bezeichnet, der jeweils gegebenenfalls mit einem oder mehreren Halogenen substituiert ist. Das Verfahren zeichnet sich dadurch aus, daß als Ausgangsstoff eine wäßrige Phase der Imidazole der allgemeinen Formel II 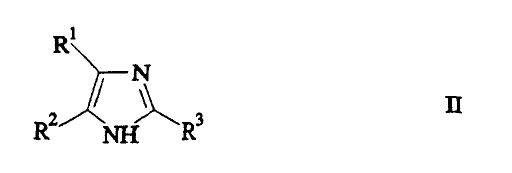 worin R¹, R² und R³ die vorgenannte Bedeutung besitzen, mit Verbindungen der allgemeinen Formel III

R⁴ - O - R⁵ III

worin
R⁴ die oben angegebene Bedeutung besitzt und
R⁵ Wasserstoff oder R⁴ bedeutet, wobei in letzterem Falle die beiden Substituenten R⁴ gleich oder verschieden sein können, eingesetzt wird und diese bei einer Temperatur von 200 bis 550 °C in Gegenwart eines Katalysators, enthaltend Oxide und/oder Phosphate eines oder mehrerer Metalle der 2., 3. und 4. Haupt- und 4. Nebengruppe des Periodensystems, in An- oder Abwesenheit von Phosphorsäure und/oder Phosphorsäureestern umgesetzt wird.

Die DE-A 16 70 293 und 21 06 877 und die DE-B 22 33 908 sowie Yoshio Ono et al., J. Chem. Soc. Chem. Commun., 9 (1995) offenbaren Verfahren zur Herstellung substituierter Imidazole, in denen Imidazole in Alkohol- und/oder Etherphasen in Gegenwart eines Katalysators zu dem gewünschten substituierten Imidazol umgesetzt werden. Diesen Verfahren ist der Nachteil gemein, daß keine wäßrigen Imidazolphasen zur Herstellung der gewünschten Imidazole verwendet werden. Laut Houben-Weyl, *Methoden der organischen Chemie,* Georg Thiele-Verlag, Stuttgart-New York, E 8c, Hetarene III/Teil 3, 4 ff., fallen Imidazole bei der Synthese üblicherweise in wäßriger Phase an.

Zur weiteren Umsetzung mit Alkoholen oder Ethern gemäß der aus dem Stand der Technik bekannten Verfahren wäre somit ein Lösungsmittelwechsel notwendig. Insbesondere bei großtechnischen Verfahren ist ein solcher Lösungsmittelwechsel mit einem erheblichen technischen und Energie-Aufwand und daher mit erheblichen Kosten verbunden.

Nur durch das Lösen in Alkoholen oder Ethern, mit denen die Imidazole umgesetzt werden sollen, kann eine für die Reaktion geeignete pumpfähige Lösung erhalten werden. Zum direkten, lösungsmittelfreien Einsatz der Imidazole wäre es nötig, die Schmelzen der Imidazole einzusetzen. Dieses ist nachteilhaft, da die Imidazole in der Regel Schmelztemperaturen zwischen 100 und 200 °C besitzen, beispielsweise besitzt 2-Methylimidazol eine Schmelztemperatur von 137 °C. Der Einsatz von Temperaturen in dem Bereich von 100 bis 200 °C ist zum einen mit einem erheblichen technischen Aufwand verbunden und führt zum anderen zu die Reaktion störenden Zersetzungsprodukten der eingesetzten Imidazole.

Die bei der Imidazolsynthese anfallende wäßrige Imidazolphase besitzt jedoch eine hervorragende Pumpfähigkeit, beispielsweise besitzt eine Imidazolphase aus .70 Gew.-% 2-Methylimidazol und 30 Gew.-% Wasser eine Schmelztemperatur von etwa 70 °C. Die aus dem Stand der Technik bekannten Verfahren besitzen den Nachteil, daß die hervorragend pumpfähigen wäßrigen Imidazolphasen nicht eingesetzt werden. Es ist weiterhin allgemein bekannt, daß bei Kondensationsreaktionen, insbesondere bei Kondensationsreaktionen, bei denen als Kondensationsprodukt Wasser entsteht, die Anwesenheit von Wasser nachteilhaft ist, weil es gemäß des Massenwirkungsgesetzes das Reaktionsgleichgewicht auf die Seite der Edukte verschiebt.

Es ist daher erfindungsgemäße Aufgabe, ein Verfahren zur Verfügung zu stellen, durch das die zuvor genannten Nachteile überwunden werden und das insbesondere den direkten Einsatz einer bei der Imidazol-Synthese anfallenden wäßrigen Imidazolphase zur Herstellung der erfindungsgemäßen substituierten Imidazole erlaubt.

Es wurde nun überraschend gefunden, daß die zuvor genannten Nachteile durch ein Verfahren gemäß des obenstehenden Wortlauts überwunden werden können.

Die Umsetzung läßt sich für den Fall der Verwendung einer wäßrigen 2-Methylimidazollösung und Methanol durch folgende Formel wiedergeben:

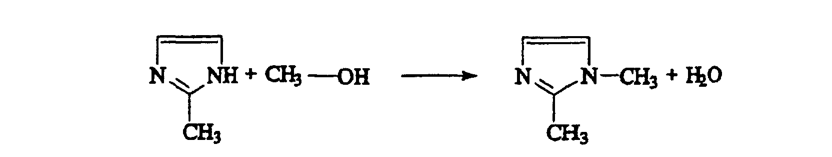

Im Vergleich zu den aus dem Stand der Technik bekannten Verfahren liefert das erfindungsgemäße Verfahren eine große Zahl von am Stickstoff substituierten Imidazolen auf einfacherem Weg und in gleichen oder besseren Ausbeuten und Reinheiten.

Erfindungsgemäß kann es vorteilhaft sein, daß der C₁- bis C₂₀-Kohlenwasserstoffrest beispielsweise einen C₃- bis C₁₂-Kohlenwasserstoffring, C₆- bis C₁₈-Aryl oder C₇- bis C₂₀-Arylalkyl bedeutet. Darüber hinaus können C₁- bis C₁₀-Kohlenwasserstoffreste bevorzugt und C₁- bis C₆-Kohlenwasserstoffreste besonders bevorzugt sein.

Erfindungsgemäße Kohlenwasserstoffreste sind vorzugsweise aliphatische Reste, wie beispielsweise C₁- bis C₂₀-Alkyl, bevorzugt C₁- bis C₁₀-Alkyl, besonders bevorzugt C₁- bis C₅-Alkyl. Unter C₁- bis C₅-Alkylreste fallen Methyl, Ethyl, n-Propyl, Isopropyl, n-Butyl, Isobutyl, tert.-Butyl, n-Pentyl, 2-Methylbutyl, 3-Methylbutyl, wobei Methyl, Ethyl, n-Propyl, Isopropyl bevorzugt und Methyl und Ethyl besonders bevorzugt sind.

Weitere erfindungsgemäß bevorzugte Kohlenwasserstoffreste sind beispielsweise aliphatische Reste, wie C₂- bis C₂₀-Alkenyl, bevorzugt C₂- bis C₁₀-Alkenyl und besonders bevorzugt C₂- bis C₅-Alkenyl. Unter C₂- bis C₅-Alkenylreste fallen beispielsweise Ethenyl, Propenyl, 1-Butenyl, 2-Butenyl, 3-Butenyl, 1,3-Butadienyl, tert.-Butenyl, 1-Pentenyl, 2-Pentenyl, 3-Pentenyl, 4-Pentenyl und Isoprenyl. Erfindungsgemäße aliphatische Reste sind beispielsweise Alkinylreste wie C₃- bis C₂₀-Alkinyl, bevorzugt C₃- bis C₁₀-Alkinyl und besonders bevorzugt C₃- bis C₅-Alkinyl. Darunter fallen beispielsweise Ethinyl, Propinyl, 1-Butinyl, 2-Butinyl, 3-Butinyl, 1-Pentinyl, 2-Pentinyl, 3-Pentinyl und 4-Pentinyl.

Weitere erfindungsgemäß bevorzugte aliphatische Reste sind Kohlenwasserstoffringe, wie C₃- bis C₁₂-Cycloalkyl, bevorzugt C₃- bis C₈-Cycloalkyl und besonders bevorzugt C₃- bis C₆-Cycloalkyl. Erfindungsgemäße C₃- bis C₆-Cycloalkylreste sind Cyclopropyl, Cyclobutyl, Cyclopentyl, Cyclohexyl, wobei Cyclobutyl, Cyclopentyl und Cyclohexyl bevorzugt und Cyclopentyl und Cyclohexyl besonders bevorzugt sind.

Die erfindungsgemäßen Kohlenwasserstoffringe können vorzugsweise eine oder mehrere Doppelbindungen und/oder Dreifachbindungen tragen, wobei beispielsweise als Ringreste mit einer Doppelbindung Cyclopropenyl, Cyclobutenyl, Cyclopentenyl, Cyclohexenyl und Cycloheptenyl und als cyclische Reste mit zwei Doppelbindungen Cyclopentadienyl und 2,4-Cyclohexadienyl in Frage kommen.

Erfindungsgemäße Arylreste sind beispielsweise Phenyl, Naphthyl und Anthracenyl, die gegebenenfalls durch einen oder mehrere C₁- bis C₈-Alkylreste und/oder Halogene substituiert sein können. Alkylarylreste sind beispielsweise Toluyl oder Xylyl, die gegebenenfalls mit einem oder mehreren Halogenen substituiert sein können. Bevorzugte erfindungsgemäße halogenierte Arylreste sind beispielsweise einfach oder mehrfach mit Halogenen, wie Fluor, Chlor, Brom, Jod, bevorzugt Fluor, Chlor und Brom, besonders bevorzugt mit Chlor substituierte Aromaten, beispielsweise 2-, 3-, 4-Chlorphenyl, 2,4-Dichlorphenyl, 2,6-Dichlorphenyl und 2,4,6-Trichlorphenyl.

Erfindungsgemäß bevorzugte, gegebenenfalls mit Halogen und/oder am Aromaten C₁- bis C₆-Alkyl substituierte Arylalkyl-Reste sind Benzyl, 1-Phenyl-ethyl und 2-Phenyl-ethyl, 1-Phenyl-propyl, 2-Phenyl-propyl, 1-Phenyl-butyl, 2-Phenyl-butyl, 3-Phenyl-butyl und 1-Phenyl-pentyl.

Weiterhin können R¹, R² und R³ voneinander unabhängig ein Halogen, wie Fluor, Chlor, Brom, Jod, bevorzugt Fluor, Chlor, Brom, besonders bevorzugt Chlor, sein.

Darüber hinaus können R¹ und R² zusammen Glieder eines gegebenenfalls mit einem oder mehreren Halogenen und/oder C₁- bis C₈-Alkyl substituierten 3 bis 20 bevorzugt 3 bis 10 und besonders bevorzugt 3 bis 6 gliedrigen Kohlenwasserstoffringes bilden, der gegebenenfalls 1, 2 oder 3 Heteroatome aus der Gruppe Stickstoff, Sauerstoff und Schwefel aufweist. Vorzugsweise sollten die Heteroatome in dem Ring so gebunden sein, daß sie keine Bindung zu einem Wasserstoffatom aufweisen, wie beispielsweise im Fall des Purins verwirklicht.

Erfindungsgemäß bevorzugt sind einfach oder mehrfach ungesättigte Kohlenwasserstoffringe, die keine Heteroatome aufweisen. Die bevorzugten durch R¹ und R² gebildeten Kohlenwasserstoffringe mit einer oder mehreren Doppelbindungen und/oder Dreifachbindungen sind beispielsweise Ringe mit einer Doppelbindung, wie Cyclopropenyl-, Cyclobutenyl-, Cyclopentenyl-, Cyclohexenyl- und Cycloheptenylringe. Bevorzugte aus R¹ und R² gebildete Kohlenwasserstoffringe mit zwei Doppelbindungen sind Cyclopentadienyl- und 2,4-Cyclohexadienylringe. Weiterhin können R¹ und R² Glieder eines Kohlenwasserstoffringes mit konjugierten Doppelbindungen sein, wobei die Zahl der konjugierten Doppelbindungen von vorzugsweise von 2 bis 7 reicht und die Ringe gemäß der Regel von Hückel entweder als aromatisches oder konjugiertes π-Elektronensystem vorliegen. Ein Imidazol mit einem derartigen Ring ist beispielsweise das Benzimidazol.

Neben Imidazol selbst können vorzugsweise folgende substituierte Imidazole als Ausgangsstoff II verwendet werden:
2-Methyl-, 2-Ethyl-, 2-n-Propyl-, 2-Isopropyl-, 2-n-Butyl-, 2-Isobutyl-, 2-tert.-Butyl- sowie 2-Phenyl-imidazol und entsprechende in 4- oder 5-Stellung substituierte Imidazole;
2-Methyl-4-ethyl-, 2,4-Dimethyl-, 2,4-Diphenyl- und entsprechende in 2,5-und 4,5-Stellung substituierte Imidazole;
2-Phenyl-4-methyl-5-ethyl-, 4-Methyl-5-phenyl-, 5-Methyl-4-phenyl-, 2,4,5-Trimethylimidazol sowie 4,5-Dimethyl-imidazol.

Erfindungsgemäß werden die Ausgangsstoffe II als wäßrige Phase vorzugsweise in einem Verhältnis von 0,1 bis 99,9, bevorzugt 50 bis 99 und besonders bevorzugt 60 bis 90 Gew.-% des Ausgangsstoffs II zu 99,9 bis 0,1, bevorzugt 50 bis 1 und besonders bevorzugt 40 bis 10 Gew.-% Wasser eingesetzt, wobei die Summe der Gew.-% von Ausgangsstoff II und Wasser jeweils 100 ergibt. Besonders bevorzugt werden die Ausgangsstoffe II in wäßrigen Phasen eingesetzt, wie sie bei der Synthese des einzusetzenden Ausgangsstoffes II anfallen. Bei den erfindungsgemäßen wäßrigen Phasen kann es sich um Lösungen, Suspensionen, Emulsionen, Aufschlämmungen etc. handeln, wobei Suspensionen und Aufschlämmungen bevorzugt und Suspensionen besonders bevorzugt sind. Ferner können in den erfindungsgemäßen wäßrigen Phasen die Löslichkeit und/oder Pumpfähigkeit des Ausgangsstoffes II steigernde Mittel, beispielsweise Seifen und oberflächenaktive Mittel, vorhanden sein.

Erfindungsgemäß bevorzugte aliphatische Reste R⁴ sind C₁- bis C₂₀-Alkyl, bevorzugt C₁- bis C₁₀-Alkyl und besonders bevorzugt C₁- bis C₅-Alkyl. Unter C₁- bis C₅-Alkylreste fallen Methyl, Ethyl, n-Propyl, Isopropyl, n-Butyl, Isobutyl, tert.-Butyl, n-Pentyl, 2-Methylbutyl, 3-Methylbutyl, wobei Methyl, Ethyl, n-Propyl, Isopropyl bevorzugt und Methyl und Ethyl besonders bevorzugt sind.

Weitere erfindungsgemäß bevorzugte aliphatische Reste R⁴ sind C₂- bis C₂₀-Alkenyl, bevorzugt C₂- bis C₁₀-Alkenyl und besonders bevorzugt C₂- bis C₅-Alkenyl. Unter C₂- bis C₅-Alkenylreste fallen beispielsweise Ethenyl, Propenyl, 1-Butenyl, 2-Butenyl, 3-Butenyl, 1,3-Butadienyl, tert.-Butenyl, 1-Pentenyl, 2-Pentenyl, 3-Pentenyl, 4-Pentenyl und Isoprenyl. Vorzugsweise steht R⁴ für 2-Buten-1-yl, 2-Penten-1-yl, 2,2-Dimethylpenten-1-yl.

Andere bevorzugte erfindungsgemäße aliphatische Reste R⁴ sind C₃- bis C₂₀-Alkinyl, bevorzugt C₃- bis C₁₀-Alkinyl und besonders bevorzugt C₃ - bis C₅-Alkinyl. Darunter fallen beispielsweise Propinyl, 1-Butinyl, 2-Butinyl, 3-Butinyl, 1-Pentinyl, 2-Pentinyl, 3-Pentinyl und 4-Pentinyl. Besonders geeignete Alkinylreste R⁴ sind Propinyl, 1,1-Dimethylpropinyl, 1-Methylpropinyl und 1-Butinyl.

Ferner bevorzugte erfindungsgemäße aliphatische Reste R⁴ sind C₃- bis C₁₂-Cycloalkyl, bevorzugt C₃- bis C₈-Cycloalkyl und besonders bevorzugt C₃bis C₆-Cycloalkyl. Erfindungsgemäße C₃- bis C₆-Cycloalkylreste R⁴ sind Cyclopropyl, Cyclobutyl, Cyclopentyl, Cyclohexyl, wobei Cyclobutyl, Cyclopentyl und Cyclohexyl bevorzugt und Cyclopentyl und Cyclohexyl besonders bevorzugt sind. Die erfindungsgemäßen aliphatischen Reste R⁴ sind auch Kohlenwasserstoffringe, die eine oder mehrere Doppelbindungen tragen, wobei beispielsweise als Reste mit einer Doppelbindung Cyclopropenyl, Cyclobutenyl, Cyclopentenyl, Cyclohexenyl und Cycloheptenyl und als cyclischer Rest mit zwei Doppelbindungen Cyclopentadienyl und 2,4-Cyclohexadienyl in Frage kommen.

Weiterhin bevorzugte erfindungsgemäße aliphatische Reste R⁴ sind Cycloalkyl-Alkyl-Reste, beispielsweise Cyclopropylmethyl, Cyclohexylmethyl.

Erfindungsgemäß bevorzugte Arylalkylreste R⁴ sind beispielsweise Benzyl, Phenylethyl, Phenylpropyl, Phenylbutyl.

Der erfindungsgemäße Rest R⁵ steht für Wasserstoff oder hat unabhängig von R⁴ die gleiche Bedeutung wie R⁴, wobei R⁵ besonders bevorzugt Wasserstoff ist.

In der Ausgangsverbindung III ist R⁵ vorzugsweise ein Rest mit einem gleichen oder geringeren Molekulargewicht als R⁴. In besonders bevorzugten Ausgangsverbindungen III stimmen die Reste R⁴ und R⁵ in der Elektrophilie der am Sauerstoff gebundenen C-Atome überein. Weiterhin besonders bevorzugt sind Ausgangsverbindungen, in denen R⁵ ein Wasserstoffatom ist.

Weiterhin kann es erfindungsgemäß bevorzugt sein, daß die Substituenten R⁴ und R⁵ in ihrem Molekulargewicht und in ihrer Struktur sowie in der Elektrophilie sich möglichst deutlich voneinander unterscheiden. Erfindungsgemäß besonders bevorzugt sind Reste R⁴ bzw. R⁵, die sich sowohl in der Elektrophilie des am Sauerstoff gebundenen C-Atoms als auch in ihrem Molekulargewicht unterscheiden. Neben dem zuvor Genannten kann es für das erfindungsgemäße Verfahren vorteilhaft sein, wenn R⁴ bzw. R⁵ in ihrem Molekulargewicht übereinstimmen, jedoch sich in der Elektronegativität der am Sauerstoff gebundenen C-Atome unterscheiden.

Beispielsweise können folgende Alkohole bzw. Ether als Ausgangsstoffe III verwendet werden: Methanol, Ethanol, n-Propanol, Isopropanol, n-Butanol, sek.-Butanol, tert.-Butanol, Pentanol, Hexanol, Heptanol, Octanol, Nonanol, Dekanol, Undekanol, Dodekanol und vorgenannten Alkoholen entsprechenden Dialkylether, Methylethylether, Methyl-n-propylether, Methylisopropylether, Methyl-n-butylether, Methyl-sek.-butylether, Methyl-tert.-butylether, Methylpentylether, Methylhexylether, Methylheptylether, Methyloctylether, Methylnonylether, Methyldecylether, Methylundecylether, Methyldodecylether sowie die analogen Ethylalkylether, Benzylpropylether, etc.

Das erfindungsgemäße Verfahren wird in Gegenwart von Katalysatoren, die Oxide und/oder Phosphate eines oder mehrerer Metalle der 2., 3. und 4. Haupt- und 4. Nebengruppe des Periodensystems der Elemente enthalten, mit oder ohne Phosphorsäure und/oder Phosphorsäureestern, durchgeführt. Bevorzugt werden die niederen Homologen der Metalle der 2., 3. und 4. Hauptgruppe und der 4. Nebengruppe des Periodensystems, insbesondere Calcium, Aluminium, Silicium, Titan, jedoch auch Zirkon und Thorium. Die Oxide und/oder Phosphate der vorgenannten Metalle können allein oder in beliebigen Gemischen miteinander der Umsetzung zugeführt werden.

Vorzugsweise werden Phosphorsäure und/oder ein oder mehrere Phosphorsäureester als Zusatzkatalysatoren zu den Oxiden und/oder Phosphaten der vorgenannten Metalle verwendet.

In den Estern kann die Phosphorsäure einfach, bevorzugt zweifach oder vorzugsweise dreifach verestert sein, gleichfalls können Mischungen dieser X Phosphorsäureester bevorzugt sein. Geeignet sind beispielsweise Pyro-, Meta - und insbesondere Orthophosphorsäure; cycloaliphatische, araliphatische, aromatische und insbesondere aliphatische Ester der Phosphorsäure, darunter insbesondere Ester mit 1 bis 12 Kohlenstoffatomen wie Triethyl-, Tri-n-butyl-, Trimethyl-, o,o-Diethyl-o-phenyl-, o-Ethyl-o-o-diphenyl-, Tricyclohexyl-, Tribenzyl-, o,o-Dimethyl-o-ethyl-, Tris-2-ethylhexyl-, Tris-β-chlorethyl-, Tris-β-butoxyethyl-, Tris-β-methoxyethyl-, Di-(2-ethylhexyl)-, Dioctyl-, Octadecyl-, Tricresyl-, o,o-Diphenyl-o-cresyl-, Trixylenyl-, Tris-(p-tert.-Butylphenyl)-, o,o-Diphenyl-o-bisphenyl-, o,o-Diphenyl-o-methyl-ester der Phosphorsäure.

Die Katalysatoren können vor ihrer Verwendung in ihrer Struktur oder Oberfläche durch physikalische und/oder chemische Behandlung, beispielsweise durch Glühen, Behandeln mit Wasserdampf, Tränken mit Säuren, beispielsweise Phosphorsäure oder Salzsäure, oder beispielsweise durch Nitrate, Formiate oder Oxalate vorgenannter Metalle, modifiziert werden. Der Katalysator kann beispielsweise durch Tränken oder Fällen auf ein Trägermaterial aufgebracht werden und gegebenenfalls in seine endgültige Oxidform durch thermische Behandlung oder Zersetzung verwandelt werden. Als Trägermaterialien kommen beispielsweise diverse Siliciumdioxide, insbesondere Kieselsäureverbindungen, wie z.B. Silikate, wie z.B. Natrium- und Calciumsilikat, Bleicherden, Tonerden, Kaolin, Zeolithe, Bimsstein, Bentonite, Kieselsäure, Kieselgel, Kieselgur etc., in Betracht. Die Trägermaterialien können gegebenenfalls noch weitere, die Reaktion nicht wesentlich beeinflussende Verbindungen anderer Elemente, beispielsweise Natrium, enthalten. Weiterhin ist es vorteilhaft, für die Reaktion einen Festbettkatalysator oder Wirbelschichtkatalysator zu verwenden.

Erfindungsgemäß bevorzugte Katalysatoren beinhalten 40 bis 99 Gew.-% SiO₂ und 1 bis 60 Gew.-% H₃PO₄, wobei 70 bis 95 Gew.-% SiO₂ und 5 bis 30 Gew.-% H₃PO₄ besonders bevorzugt sind. Ein weiterer erfindungsgemäß bevorzugter Katalysator beinhaltet 30 bis 99 Gew.-% Al₂O₃ und 1 bis 70 Gew.-% SiO₂, wobei 70 bis 90 Gew.-% Al₂O₃ und 10 bis 30 Gew.-% SiO₂ besonders bevorzugt sind. Die Summe aller Bestandteile der erfindungsgemäßen Katalysatoren ergibt jeweils 100%.

Neben der bevorzugten Durchführung des erfindungsgemäßen Verfahrens in einem Festbettreaktor mit einem Festbettkatalysator ist es gleichfalls möglich, das erfindungsgemäße Verfahren in anderen Reaktortypen mit anderen Katalysatoraufbereitungen, beispielsweise in einem Wirbelschichtreaktor mit einem entsprechend dafür aufgearbeiteten Katalysator durchzuführen.

Das erfindungsgemäße Verfahren kann vorzugsweise wie folgt durchgeführt werden: Ein Gemisch der Ausgangsstoffe II und III wird auf den auf die Reaktionstemperatur erhitzten Katalysator in einem Röhren- oder Wirbelschicht- oder Festbettreaktor, gegebenenfalls in einer Inertgasatmosphäre, beispielsweise aus Stickstoff oder Argon, geleitet. Das Reaktionsprodukt kann gegebenenfalls durch bekannte Verfahren, z.B. Chromatografie oder fraktionierte Destillation, aufgearbeitet werden. Das erfindungsgemäße Verfahren wird bevorzugt als Gasphasenreaktion betrieben.

Ein besonders bevorzugtes erfindungsgemäßes Verfahren verwendet als Ausgangsstoffe II die pumpfähigen wäßrigen Phasen der zu substituierenden Imidazole. In einem besonders bevorzugten erfindungsgemäßen Verfahren wird 2-Methylimidazol als pumpfähige wäßrige Phase als Ausgangsstoff II eingesetzt. Als Ausgangsstoff II sind insbesondere die wäßrigen Imidazolphasen bevorzugt, die direkt aus der Imidazolsynthese stammen. In den zuvor genannten Varianten des erfindungsgemäßen Verfahrens kann es bevorzugt sein, die wäßrige Imidazolphase soweit zu erwärmen, daß ihre Pumpfähigkeit ein Optimum erreicht. In einem weiteren erfindungsgemäß besonders bevorzugten Verfahren wird eine im Temperaturbereich von 60 bis 80 °C verflüssigte wäßrige Phase aus 70 Gew.-% 2-Methylimidazol und 30 Gew.-% Wasser verwendet.

Die Umsetzung wird in einem Temperaturbereich von 200 bis 550, vorzugsweise zwischen 250 und 450 und besonders bevorzugt in einem Bereich von 280 bis 420 °C durchgeführt.

Die nach dem erfindungsgemäßen Verfahren hergestellten Verbindungen sind insbesondere als Vor- und Zwischenprodukte sowie als Katalysatoren für die Herstellung von Farbstoffen, Textilhilfsmitteln, Arzneimittelwirkstoffen, Kunststoffen und Pflanzenschutzmitteln geeignet.

Die Erfindung wird nachfolgend anhand von Beispielen erläutert, wobei die in den Beispielen genannten Teile Gewichtsteile bedeuten.

### BEISPIELE

### Beispiel 1

In einem kontinuierlich betriebenen Festbettreaktor wird über den Festbettkatalysator (80 Gew.-% SiO₂, 20 Gew.-% H₃OP₄) bei 330 °C stündlich ein Gemisch von 10,6 Teilen 2-Methylimidazol, 5 Teilen Wasser und 12,4 Teilen Methanol geleitet. In bestimmten Zeitabständen wird dem Rohaustrag eine Probe entnommen und gaschromatografisch analysiert. Der Umsatz liegt bei 100%, die Ausbeute bei 96%.

### Beispiel 2

In einem kontinuierlich betriebenen Festbettreaktor wird über den Festbettkatalysator (80 Gew.-% Al₂O₃, 20 Gew.-% SiO₂) bei 350 °C stündlich ein Gemisch von 10,6 Teilen 2-Methylimidazol, 5 Teilen Wasser und 25,6 Teilen Methanol geleitet. In bestimmten Zeitabständen wird dem Rohaustrag eine Probe entnommen und gaschromatografisch analysiert. Analog zu Beispiel 1 bestimmt, betragen der Umsatz 98% und die Ausbeute 94%.

## Patentansprüche

1. Verfahren zur Herstellung von substituierten Imidazolen der allgemeinen Formel I 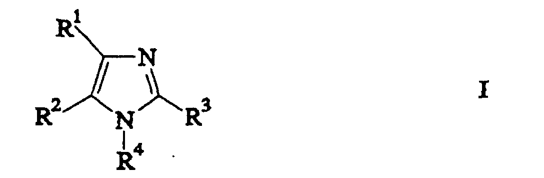 worin
R¹, R² und R³ gleich oder verschieden sein können und jeweils Wasserstoff, Halogen, NO₂, CN oder einen gegebenenfalls mit einem oder mehreren Halogenen substituierten C₁- bis C₂₀-Kohlenwasserstoffrest bedeuten oder R¹ und R² zusammen Glieder eines gegebenenfalls mit einem oder mehreren Halogenen und/oder C₁- bis C₈-Alkyl substituierten 3 bis 20 gliedrigen Kohlenwasserstoffringes bilden, der gegebenenfalls 1, 2 oder 3 Heteroatome aus der Gruppe Stickstoff, Sauerstoff und Schwefel aufweist, und
R⁴ einen C₁- bis C₂₀-Kohlenwasserstoffrest bezeichnet, der jeweils gegebenenfalls mit einem oder mehreren Halogenen substituiert ist, **dadurch gekennzeichnet, daß** als Ausgangsstoff eine wäßrige Phase der Imidazole der allgemeinen Formel II 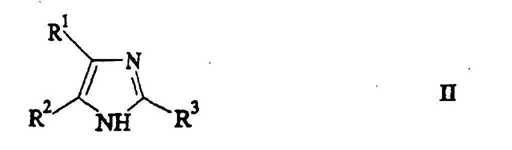 worin R¹, R² und R³ die
vorgenannte Bedeutung besitzen, mit Verbindungen der allgemeinen Formel III
R⁴ - O - R⁵ III
worin
R⁴ die oben angegebene Bedeutung besitzt und
R⁵ Wasserstoff oder R⁴ bedeutet, wobei in letzterem Falle die beiden Substituenten R⁴ gleich oder verschieden sein können,
eingesetzt wird und diese bei einer Temperatur von 200 bis 550 °C in Gegenwart eines Katalysators, enthaltend Oxide und/oder Phosphate eines oder mehrerer Metalle der 2., 3. und 4. Haupt- und 4. Nebengruppe des Periodensystems, in An- oder Abwesenheit von Phosphorsäure und/oder Phosphorsäureestern umgesetzt wird.

2. Verfahren nach Anspruch 1, **dadurch gekennzeichnet, daß** der C₁- bis C₂₀-Kohlenwasserstoffrest C₁ bis C₂₀-Alkyl, C₂- bis C₂₀-Alkenyl, C₃- bis C₂₀-Alkinyl, einen C₃- bis C₁₂-Kohlenwasserstoffring, C₆- bis C₁₈-Aryl oder C₇- bis C₂₀-Arylalkyl bedeutet.

3. Verfahren nach Anspruch 1 oder 2, **dadurch gekennzeichnet, daß** in einem Temperaturbereich von 250 bis 450 °C mit einem Katalysator, enthaltend die Oxide des Siliciums und/oder Aluminiums, in An- oder Abwesenheit von Phosphorsäure umgesetzt wird.

4. Verfahren nach einem der Ansprüche 1 bis 3, **dadurch gekennzeichnet, daß** in Gegenwart eines Katalysators, enthaltend 40 bis 99 Gew.-% SiO₂ und 1 bis 60 Gew.-% H₃PO₄, umgesetzt wird.

5. Verfahren nach einem der Ansprüche 1 bis 3, **dadurch gekennzeichnet, daß** in Gegenwart eines Katalysators, enthaltend 30 bis 99 Gew.-% Al₂O₃ und 1 bis 70 Gew.-% SiO₂, umgesetzt wird.

6. Verfahren nach einem der Ansprüche 1 bis 5, **dadurch gekennzeichnet, daß** eine wäßrige Imidazolphase mit einem Verhältnis von 0,1 bis 99,9 Gew.-% Imidazol der Formel II zu 0,1 bis 99,9 Gew.-% Wasser umgesetzt wird.

7. Verfahren nach einem der Ansprüche 1 bis 6, **dadurch gekennzeichnet, daß** mit einem Festbettkatalysator umgesetzt wird.

8. Verfahren nach einem der Ansprüche 1 bis 7, **dadurch gekennzeichnet, daß** die Umsetzung in Gasphase erfolgt.

9. Verfahren nach einem der Ansprüche 1 bis 8, **dadurch gekennzeichnet, daß** kontinuierlich umgesetzt wird.

10. Verfahren nach einem der Ansprüche 1 bis 9, **dadurch gekennzeichnet, daß** als Ausgangsstoff das wäßrige Produktgemisch aus der Herstellung von Imidazolen der allgemeinen Formel II, vorzugsweise 2-Methylimidazol, eingesetzt wird.

## Claims

1. A process for preparing substituted imidazoles of the general formula I 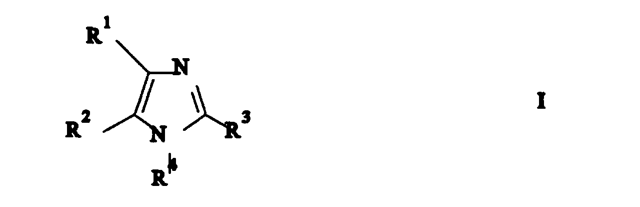 where
R¹, R² and R³ are identical or different and are each hydrogen, halogen, NO₂ CN or a C₁-C₂₀-hydrocarbon radical which is unsubstituted or substituted by one or more halogens or R¹ and R² together are members of a 3- to 20-membered hydrocarbon ring which is unsubstituted or substituted by one or more halogens and/or C₁-C₈-alkyl groups and which may contain 1, 2 or 3 heteroatoms from the group consisting of nitrogen, oxygen and sulfur, and
R⁴ is a C₁-C₂₀-hydrocarbon radical which is unsubstituted or substituted by one or more halogens, which comprises using as starting material an aqueous phase of the imidazoles of the general formula II 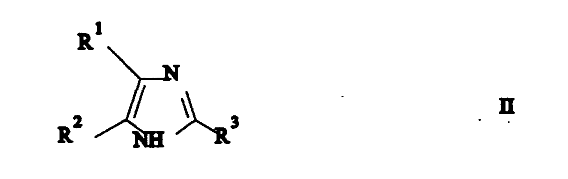 where R¹, R² and R³ are
as defined above with compounds of the general formula III
R⁴-O-R⁵ III
where
R⁴ is as defined above and
R⁵ is hydrogen or R⁴, it being possible in the latter case for the two R⁴ substituents to be identical or different, and
reacting it at from 200 to 550°C in the presence of a catalyst comprising oxides and/or phosphates of one or more metals of the 2nd, 3rd and 4th main group and the 4th subgroup of the Periodic Table in the presence or absence of phosphoric acid and/or phosphoric esters.

2. A process as claimed in claim 1 wherein the C₁-C₂₀-hydrocarbon is C₁-C₂₀-alkyl, C₂-C₂₀-alkenyl, C₃-C₂₀-alkynyl, a C₃-C₁₂-hydrocarbon ring, C₆-C₁₈-aryl or C₇-C₂₀-aralkyl.

3. A process as claimed in claim 1 or 2 wherein the reaction is carried out at from 250 to 450°C in the presence or absence of phosphoric acid using a catalyst comprising silicon oxides and/or aluminum oxides.

4. A process as claimed in any of claims 1 to 3 wherein the reaction is carried out in the presence of a catalyst comprising from 40 to 99% by weight of SiO₂ and from 1 to 60% by weight of H₃PO₄.

5. A process as claimed in any of claims 1 to 3 wherein the reaction is carried out in the presence of a catalyst comprising from 30 to 99% by weight of Al₂O₃ and from 1 to 70% by weight of SiO₂.

6. A process as claimed in any of claims 1 to 5 wherein the reaction is carried out using an aqueous imidazole phase having a ratio of from 0.1 to 99.9% by weight of imidazole of the formula II to from 0.1 to 99.9% by weight of water.

7. A process as claimed in any of claims 1 to 6 wherein the reaction is carried out using a fixed-bed catalyst.

8. A process as claimed in any of claims 1 to 7 wherein the reaction is carried out in the gas phase.

9. A process as claimed in any of claims 1 to 8 wherein the reaction is carried out in a continuous manner.

10. A process as claimed in any of claims 1 to 9 wherein the starting material used is the aqueous product mixture from the preparation of imidazoles of the general formula II, preferably 2-methylimidazole.

## Revendications

1. Procédé de préparation d'imidazoles substitués de formule générale I 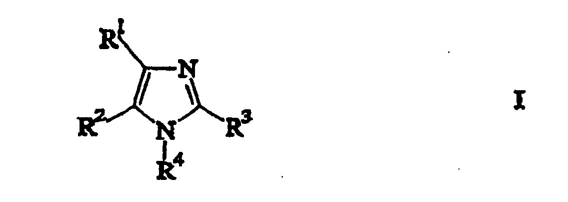 dans laquelle
R¹, R² et R³ peuvent être identiques ou différents et représentent chacun un atome d'hydrogène, un atome d'halogène, NO₂, CN ou un groupe hydrocarboné en C₁ à C₂₀ éventuellement substitué par un ou plusieurs atomes d'halogène, ou bien R¹ et R² forment ensemble des éléments d'un cycle hydrocarboné de 3 à 20 éléments, éventuellement substitué par un ou plusieurs atomes d'halogène et/ou par un groupe alkyle en C₁ à C₈, qui présente éventuellement 1, 2 ou 3 hétéroatomes du groupe formé par les atomes d'azote, d'hydrogène et de soufre, et
R⁴ représente un groupe hydrocarboné en C₁ à C₂₀ qui est à chaque fois éventuellement substitué par un ou plusieurs atomes d'halogène,
**caractérisé en ce que** l'on met en oeuvre, en tant que produit de départ, une phase aqueuse des imidazoles de formule générale II 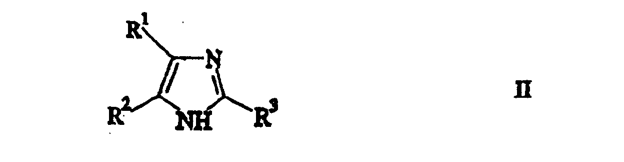 dans laquelle
R¹, R² et R³ ont la signification susmentionnée, avec des composés de formule générale III
R⁴-O-R⁵ III
dans laquelle
R⁴ a la signification susmentionnée et
R⁵ représente un atome d'hydrogène ou R⁴, auquel cas les deux substituants R⁴ peuvent être identiques ou différents,
et on la fait réagir à une température allant de 200°C à 550°C en présence d'un catalyseur contenant des oxydes et/ou des phosphates d'un ou de plusieurs métaux des groupes principaux 2, 3 et 4 et du sous-groupe 4 de la Classification Périodique des Eléments, en présence ou en l'absence d'acide phosphorique et/ou d'esters d'acide phosphorique.

2. Procédé selon la revendication 1, **caractérisé en ce que** le groupe hydrocarboné en C₁ à C₂₀ est un groupe alkyle en C₁ à C₂₀, un groupe alcényle en C₂ à C₂₀, un groupe alcynyle en C₃ à C₂₀, un cycle hydrocarboné en C₃ à C₁₂, un groupe aryle en C₆ à C₁₈ ou un groupe arylalkyle en C₇ à C₂₀.

3. Procédé selon la revendication 1 ou 2, **caractérisé en ce que** l'on réalise la réaction dans une gamme de température allant de 250°C à 450°C avec un catalyseur contenant les oxydes du silicium et/ou de l'aluminium, en présence ou en l'absence d'acide phosphorique.

4. Procédé selon l'une quelconque des revendications 1 à 3, **caractérisé en ce que** l'on réalise la réaction en présence d'un catalyseur contenant 40% à 99% en poids de SiO₂ et 1% à 60% en poids de H₃PO₄.

5. Procédé selon l'une quelconque des revendications 1 à 3, **caractérisé en ce que** l'on réalise la réaction en présence d'un catalyseur contenant 30% à 99% en poids de Al₂O₃ et 1% à 70% en poids de SiO₂.

6. Procédé selon l'une quelconque des revendications 1 à 5, **caractérisé en ce que** l'on fait réagir une phase aqueuse d'imidazole ayant un rapport de 0,1% à 99,9% en poids d'imidazole de formule II à 0,1% à 99,9% en poids d'eau.

7. Procédé selon l'une quelconque des revendications 1 à 6, **caractérisé en ce que** l'on réalise la réaction avec un catalyseur en lit fixe.

8. Procédé selon l'une quelconque des revendications 1 à 7, **caractérisé en ce que** l'on réalise la réaction en phase gazeuse.

9. Procédé selon l'une quelconque des revendications 1 à 8, **caractérisé en ce que** l'on réalise la réaction en continu.

10. Procédé selon l'une quelconque des revendications 1 à 9, **caractérisé en ce que** l'on met en oeuvre, en tant que produit de départ, le mélange aqueux de produits issus de la préparation d'imidazoles de formule générale II, de préférence le 2-méthylimidazole.
